# EUROPEAN PATENT SPECIFICATION

(11) **EP 2 026 782 B1**
(45) Date of publication and mention of the grant of the patent: **14.11.2012**
(21) Application number: 07776756.4
(22) Date of filing: 03.05.2007
(51) Int. Cl.: C07C 211/09, A61K 31/132

(54) **N1,N4-BIS(BUTA-1,3-DIENYL)BUTANE-1,4-DIAMINE PHARMACEUTICAL COMPOSITIONS AND USES THEREOF**
PHARMAZEUTISCHE ZUSAMMENSETZUNGEN MIT N1,N4-BIS(BUTA-1,3-DIENYL)BUTAN-1,4-DIAMIN UND IHRE VERWENDUNG
COMPOSITIONS PHARMACEUTIQUES DE LA N1,N4-BIS(BUTA-1,3-DIÉNYL)BUTANE-1,4-DIAMINE ET PROCÉDÉS D'UTILISATION ET DE FABRICATION DE CELLES-CI

(30) Priority: 03.05.2006 US 797142 P
(43) Date of publication of application: 25.02.2009
(73) Proprietor: WISCONSIN ALUMNI RESEARCH FOUNDATION, Madison, WI 53707-7365 (US)
(72) Inventor: BASU, Hirak, S., Madison, WI 53717 (US); CHURCH, Dawn, R., Stoughton, WI 53589 (US); WOSTER, Patrick, M., Canton, MI 48188 (US); WILDING, George, Verona, WI 53593 (US)
(74) Representative: Gosnall, Toby
(86) International application number: PCT/US2007/010864
(87) International publication number: WO 2007/130589

(56) References cited:
- QUEMENER V ET AL: "POLYAMINE DEPRIVATION: A NEW TOOL IN CANCER TREATMENT" ANTICANCER RESEARCH, HELENIC ANTICANCER INSTITUTE, ATHENS,, GR, vol. 14, no. 2A, March 1994 (1994-03), pages 443-448, XP008014124 ISSN: 0250-7005
- AGOSTINELLI E ET AL: "Sensitization of human colon adenocarcinoma clles (LoVo) to reactive oxygen species by a lysosomotropic compound" INTERNATIONAL JOURNAL OF ONCOLOGY, vol. 29, no. 4, October 2006 (2006-10), pages 947-955, XP002452763
- DATABASE BIOSIS [Online] BIOSCIENCES INFORMATION SERVICE, PHILADELPHIA, PA, US; April 2004 (2004-04), XU HINGXIU ET AL: "H-pylori-induced gastric epithelial cell apoptosis and DNA damage is caused by polyamine oxidation" XP002452779 Database accession no. PREV200600083292 & GASTROENTEROLOGY, vol. 126, no. 4, Suppl. 2, April 2004 (2004-04), page A59, DIGESTIVE DISEASE WEEK/105TH ANNUAL MEETING OF THE AMERICAN-GASTROENTEROLOGICAL-ASSOCIATION; NEW ORLEANS, LA, USA; MAY 16 20, 2004 ISSN: 0016-5085
- CASERO R A ET AL: "The role of polyamine catabolism in anti-tumour drug response" BIOCHEMICAL SOCIETY TRANSACTIONS, vol. 31, no. 2, 2003, pages 361-365, XP002452764
- CHATURVEDI R ET AL: "Induction of Polyamine Oxidase 1 by Helicobacter pylori Cause Macrophage Apoptosis by Hydrogen Peroxide Release and Mitochondrial Membrane Depolarisation" THE JOURNAL OF BIOLOGICAL CHEMISTRY, vol. 279, no. 38, 2004, pages 40161-40173, XP002452765
- BASU H ET AL: "An inhibitor of acetyl polyamine oxidase specifically blocks androgen induced oxidative stress and prevents occurrence of prostate cancer in TRansgenic Adenocarcinoma of Mouse Prostate (TRAMP)" EUROPEAN JOURNAL OF CANCER SUPPLEMENTS, vol. 4, no. 12, November 2006 (2006-11), page 40, XP002452791
- BASU H S ET AL: "Novel prodrugs that are activated by oxidative stress in prostate cancer" PROCEEDINGS OF THE AMERICAN ASSOCIATION FOR CANCER RESEARCH ANNUAL MEETING APRIL 2005, vol. 46, April 2005 (2005-04), pages 149-150, XP001536695
- QUEMENER V ET AL: "Implication des polyamines dans les processus prolifératifs malins: effets anticancereux d'une carence en polyamines" BULLETIN DE L'ACADEMIE NATIONALE DE MEDICINE, vol. 178, no. 8, November 1994 (1994-11), pages 1591-1605, XP009090071
- QUEMENER V ET AL: "Implication des polyamines dans les processus prolifératifs malins" ANNALES DE GASTROENTEROLOGIE ET D'HEPATOLOGIE, vol. 31, no. 3, 1995, pages 181-189, XP009090070

## Description

### BACKGROUND OF THE INVENTION

Advanced hormone refractory metastatic prostate cancer is the second leading cause of cancer deaths among men living in the U.S. It is determined that in 2006, over 27,000 U.S. men will died of metastatic prostate cancer. Currently, most chemotherapeutic agents used in clinics are limited in treating the disease. Thus, there exists substantial need for new therapeutic agents to treat metastatic prostate cancer.

At the time of initial diagnosis, most prostate cancer patients have androgen dependent tumors that regress quickly after surgery, radiation and androgen ablation therapy. However, the cancer recurs a few years later in a considerable percentage of such patients. The recurrence shows up in the form of advanced hormone refractory metastatic disease. Currently, there exists no effective therapy for treating or preventing this disease, particularly in an advanced state. As such, there exists a substantially urgent need for developing drugs to reduce the recurrence and progression of prostate cancer.

It has been theorized that oxidative stress in prostate tissue is a major contributor to prostate cancer occurrence and progression. Hence, there further exists an urgent need to discover and develop drugs that therapeutically reduce oxidative stress.

Published epidemiological and biochemical evidence suggests that oxidative stress in prostate tissue is one of the major contributors to prostate cancer occurrence and progression and that antioxidants can reduce prostate carcinogenesis. It is further known that androgen is one of the major inducers of ROS in normal and malignant prostate cells. (See Wilding, G., Endocrine Control of Prostate, Cancer, Cancer Surveys, 23:43-62 (1995)). It is also known that within the polyamine catabolic pathway, recycling of the acetyl polyamine oxidase ("APAO") enzyme is a major source of ROS production. (See Cohen, S.S., A Guide to the Polyamines, Oxford Univ. Press, Oxford UK: 296-319 (1998); Schwartz, B et al, A New Model for Disruption of the Ornithine Decarboxylase Gene, SPE1, In Saccharomyces Cerevisiae Exhibits Growth Arrest and Genetic Instability at the MAT Locus, Biochem J., Nov 15:312 (Pt. 1):83-90 (1995); Schipper RG et al, Antitumor Activity of the Polyamine Analog N(1),N(11)-diethylnorspermine Against Human Prostate Carcinoma Cells, The Prostate, 44(4):313-21 (2000); Casero, RA et al, The Role of Polyamine Catabolism in Anti-tumour Drug Response, Biochem. Soc. Trans., Apr:31(2):361-5 (2003); Ha, HC et al, The Role of Polyamine Catabolism in Polyamine Analogue-Induced Programmed Cell Death, Proc. Natl. Acad. Sci. USA, 94(21):11557-62 (1997); and, Bey, P et al, N-2,3-Butadienyl-1,4-butanediamine Derivatives: Potent Irreversible Inactivators of Mammalian Polyamine Oxidase, J. Med. Chem, 28(1):1-2 (1985)).

### SUMMARY OF THE INVENTION

An aspect of the invention is a compound which is N,N'-bis(2,3-butadienyl)-1,4-butanediamine or a salt or solvate thereof for use in the treatment of cancer by inhibiting acetyl polyamine oxidase in the prostate of a human male. In an exemplary embodiment, the acetyl polyamine oxidase is inhibited by at least 50% as compared to an untreated human male.

Another aspect of the invention is N,N'-bis(2,3-butadienyl)-1,4-butanediamine or a pharmaceutically suitable salt or solvate thereof, for use in prophylactically treating cancer of the prostate in a male human.

In an exemplary embodiment of the above method, the compound is used in a therapeutic amount which is an amount sufficient to prevent or reduce the occurrence and/or recurrence of prostate cancer as compared to an untreated human male control with or without previously diagnosed prostate cancer.

Another aspect of the invention is the compound for use in treating cancer in the prostate of a human male.

In an exemplary embodiment of the above method, the compound is used in a therapeutic amount which is an amount sufficient to stop or reduce the progression, morbidity and/or mortality due to prostate cancer.

In an exemplary embodiment of any of the above methods, the salt is an acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, mitrate, pamoate, pantothenate, phosphate, diphosphate, polygalacturonate, salicylate, stearate, subacetate, succinate, sulfate, tannate, tartrate, teoclate, or triethiodide.

In another exemplary embodiment of any of the above methods, the salt is a dihydrochloride salt.

In another exemplary embodiment of any of the above methods, the therapeutic amount is in the range of about 1-100 mg/kg_{BW}, and the therapeutic amount is dosed in the range of bi-weekly to daily.

In another exemplary embodiment of any of the above methods, the therapeutic amount is in the range of about 10-40 mg/kg_{BW}, and the therapeutic amount is dosed weekly.

In another exemplary embodiment of any of the above methods, the therapeutic amount is around 25 mg/kg_{BW}, and the therapeutic amount is dosed bi-weekly.

In an exemplary embodiment of the oral pharmaceutical composition, the salt is an acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, mitrate, pamoate, pantothenate, phosphate, diphosphate, polygalacturonate, salicylate, stearate, subacetate, succinate, sulfate, tannate, tartrate, teoclate, or triethiodide.

In an exemplary embodiment of the oral pharmaceutical composition, the composition is in the form being an uncoated tablet, coated tablet, hard gelatin capsule, soft gelatin capsule, powder, capsule, pellet, solution, suspension, elixir or emulsion.

Another aspect of the invention is a method of determining oxidative stress in human tissue comprising the step or act of measuring *ex vivo* a ratio of oxidized 2',7'-dichlorodihydrofluorescein diacetate fluorescence:DNA fluorescence.

Another aspect of the invention is a method of determining oxidative stress in human tissue comprising the step or act of measuring *ex vivo* a ratio of oxidized hydroethidine fluorescence:DNA fluorescence.

Another aspect of the invention is a method of determining oxidative stress in human male prostate tissue comprising the step or act of measuring a ratio of oxidized 2',7'-dichlorodihydrofluorescein diacetate fluorescence:DNA fluorescence in *vivo.*

In an exemplary embodiment of the above methods, the human tissue is human male prostate tissue derived from a tumor biopsy.

In another exemplary embodiment of the above methods, the human tissue is taken from a tumor biopsy from a part of the body other than the prostate.

Another aspect of the invention is a method of treating cancer in the prostate of a male dog comprising the step or act of administering a therapeutic amount of N,N'-bis(2,3-butadienyl)-1,4-butanediamine or a pharmaceutically suitable salt or solvate thereof to the dog.

Another aspect of the invention is a method of reducing the concentration of reactive oxygen species in human tissue comprising the step or act of administering a therapeutic amount of N,N'-bis(2,3-butadienyl)-1,4-butanediamine or a pharmaceutically suitable salt or solvate thereof to the human.

In an exemplary embodiment of the above method, the therapeutic amount is an amount sufficient to lower the concentration of one or more reactive oxygen species in the prostate by at least 50% as compared to the concentration of reactive oxygen species in untreated human tissue.

In an exemplary embodiment of the above method, the reactive oxygen species are one or more of hydrogen peroxide, superoxide, hydroxyl radical and nitric oxide.

Another aspect of the invention is a reagent kit for measuring the concentration of reactive oxygen species *ex vivo* or *in vivo* in a mammal cell, organ or biopsy comprising a first component comprising a hydroethidine dye, and, a second component comprising a live cell DNA stain.

In an exemplary embodiment of the above reagent kit, the live cell DNA stain comprises:

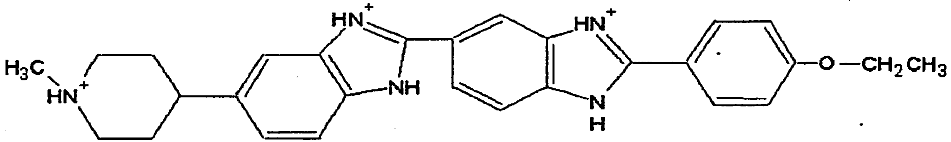

Another aspect of the invention is a method of using the above kit to measure the concentration of reactive oxygen species *ex vivo* in tissue derived from mammal cells, organs or biopsies comprising the steps or acts of dyeing a first tissue with the hydroethidine dye to produce a first number of fluorescence units, dyeing a second tissue with the live cell DNA stain to produce a second number of fluorescence units, and, normalizing the first number of fluorescence units to the second number of fluorescence units to quantify the concentration of reactive oxygen species.

In an exemplary embodiment of the above method, the live cell DNA comprises:

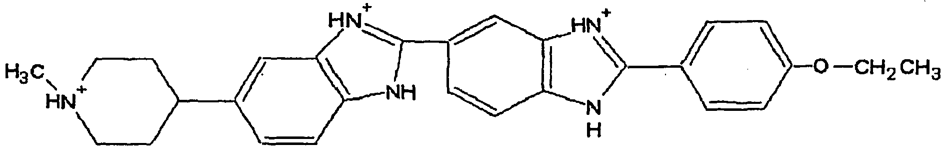

Another aspect of the invention is a reagent kit for measuring the concentration of reactive oxygen species *ex vivo* or *in vivo* in a mammal cell, organ or biopsy comprising a first component comprising a 2',7'-dichlorodihydrofluorescein diacetate dye, and, a second component comprising a live cell DNA stain.

In an exemplary embodiment of the above reagent kit, the live cell DNA comprises:

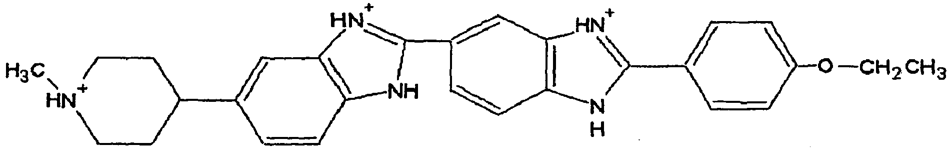

Another aspect of the invention is a method of using the above reagent kit to measure the concentration of reactive oxygen species *ex vivo* in tissue derived from mammal cells, organs or biopsies comprising the steps or acts of dyeing a first tissue with the 2',7'-dichlorodihydrofluorescein diacetate dye to produce a first number of fluorescence units, dyeing a second tissue with the live cell DNA stain to produce a second number of fluorescence units, and, normalizing the first number of fluorescence units to the second number of fluorescence units to quantify the concentration of reactive oxygen species.

In an exemplary embodiment of the above method, the live cell DNA comprises:

### BRIEF DESCRIPTION OF THE EXEMPLARY DRAWINGS

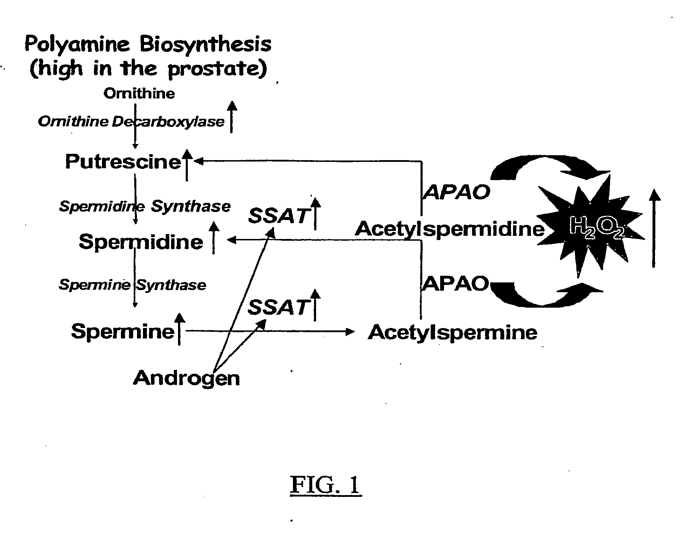

FIG. 1 is a schematic illustration of polyamine metabolism in terms of androgen induced SSAT induction in prostate cells causing polyamine oxidation and ROS production.

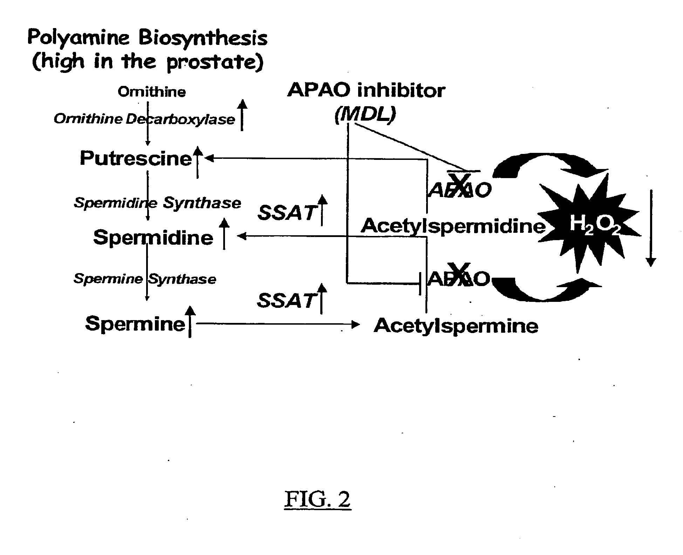

FIG. 2 is a theoretical schematic illustration of the N,N'-bis(2,3-butadienyl)-1,4-butanediamine compound of the instant invention acting as an inhibitor of acetyl polyamine oxidase (APAO) and blocking androgen induced ROS production in prostate cells.

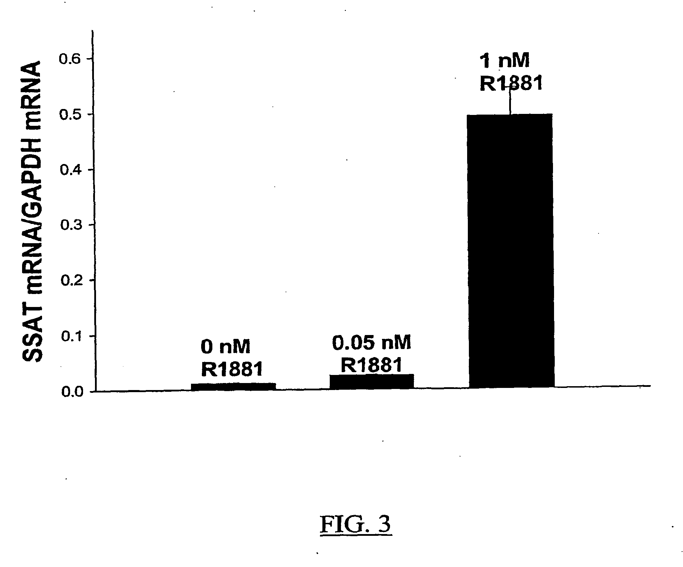

FIG. 3 is a graphical illustration showing a plot of qRT-PCR quantitation of SSAT mRNA level normalized to glyceraldehyde-3-phosphodehydrogenase mRNA levels in untreated LNCaP human prostate cancer cells, and cells treated with 0.05 nM or 1.0 nM R1881 for 96 hours, whereby the QRT-PCR data shows that 1 nM R1881 (a synthetic androgen analog) treatment increases SSAT mRNA levels in LNCaP cells.

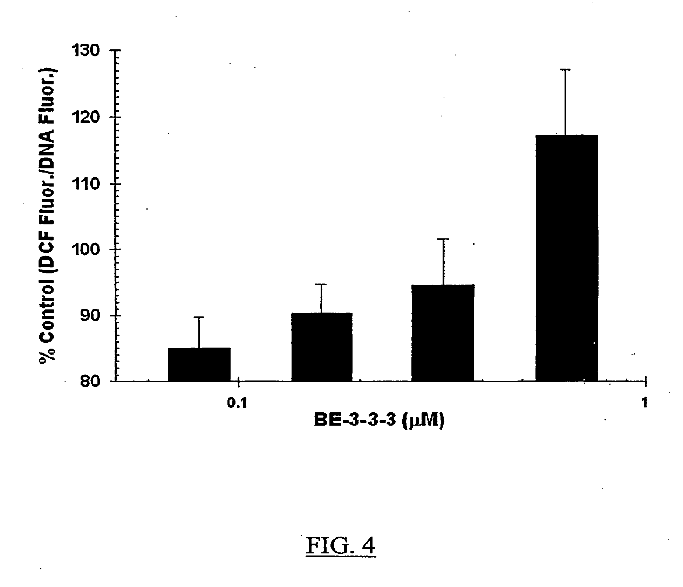

FIG. 4 is a graphical illustration showing a plot of DCF fluorescence/DNA fluorescence in LNCaP cells at 72 hours treatment with increasing non-androgenic compound bisethyl norspermine ("BE-3-3-3") concentration in DU-145 human prostate cancer cells, whereby the data are normalized to percent of control untreated cells, and whereby BE-3-3-3 (a known inducer of SSAT) increases oxidative stress in DU-145 cells.

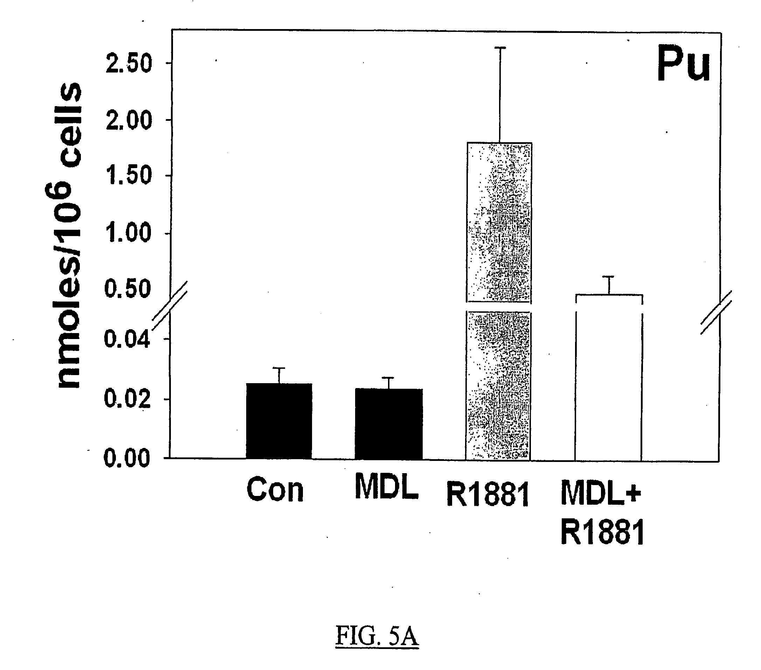

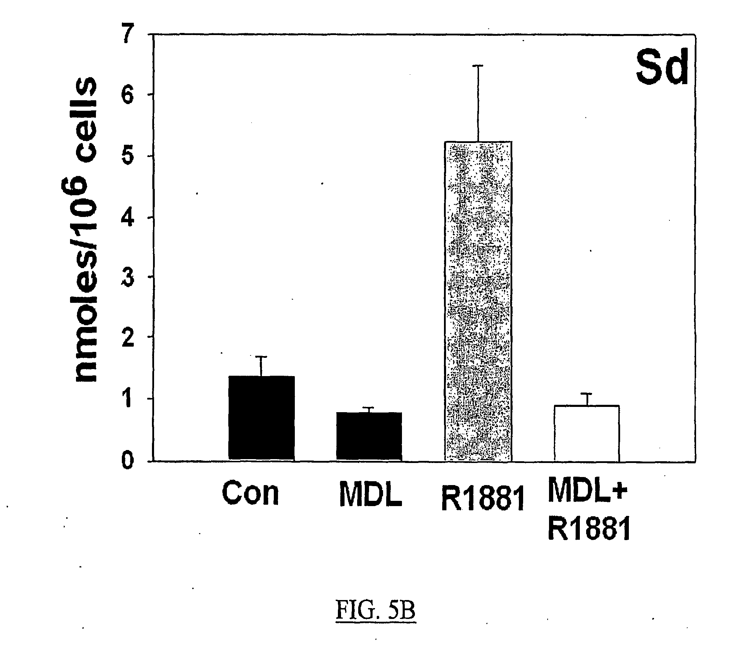

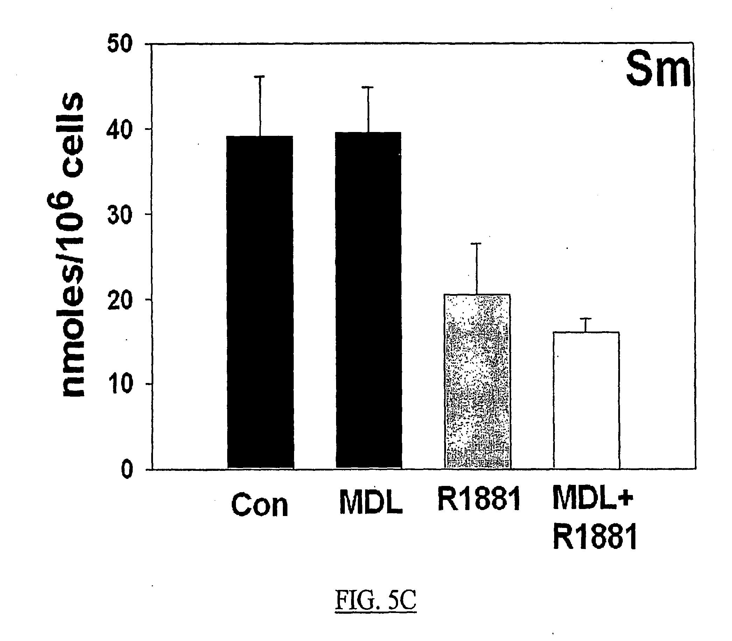

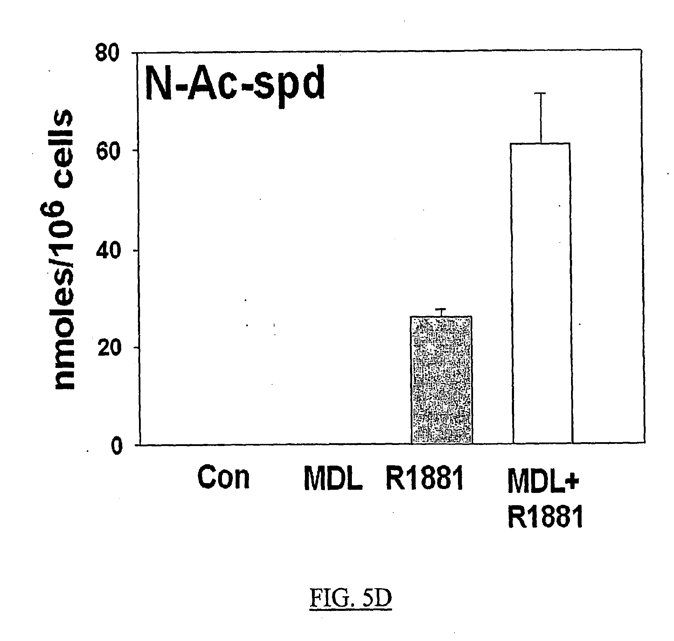

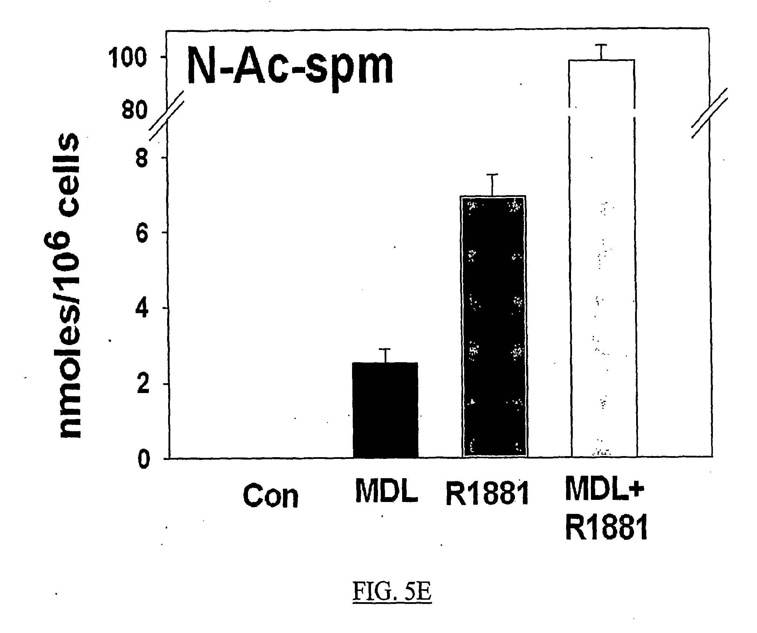

FIGS. 5A-5E are a graphical illustrations showing plots of putrescine (Pu), spermidine (Sd), spermine (Sm), N-acetyl spermidine (N-Ac-Sd) and N-acetyl spermine (N-Ac-Sm) levels in LNCaP human prostate cells treated with 25 µg N,N'-bis(2,3-butadienyl)-1,4-butanediamine compound for 120 hours, and, with and without treatment with 1 nM R1881 for 96 hours, and cells pretreated for 24 hours with 25 µM N,N'-bis(2,3-butadienyl)-1,4-butanediamine compound followed by treatment with 1 nM R1881 for 96 hours, whereby polyamine levels are expressed in nmoles/10⁶ cells and were determined using the HPLC method (see Kabra, PM et al, Solid-Phase Extraction and Determination of Dansyl Derivatives of Unconjugated and Acetylated Polyamines by Reverse-Phase Liquid Chromatography: Improved Separation Systems for Polyamines in Cerebrospinal Fluid, Urine and Tissue, J. Chromatogr., 1986;380(1):19-32) using commercially available standards, and whereby the data shows polyamine and acetyl polyamine levels in LNCaP cells treated with 1nM R1881 ± 25 µM MDL 72,527.

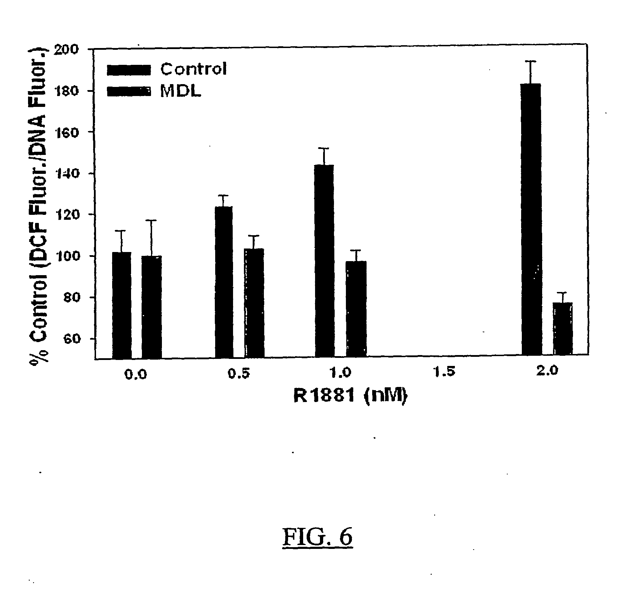

FIG. 6 is a graphical illustration showing a plot of DCF fluorescence/DNA fluorescence in LNCaP cells expressed as a percentage of control untreated cells treated with increasing R1881 concentration, whereby the data shows that pretreatment with MDL 72,527 effectively blocks androgen induced ROS production in LNCaP cells.

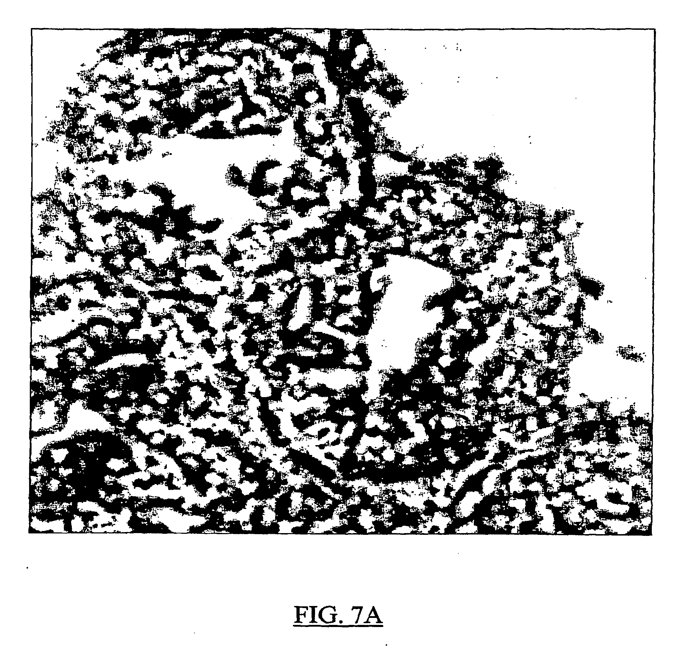

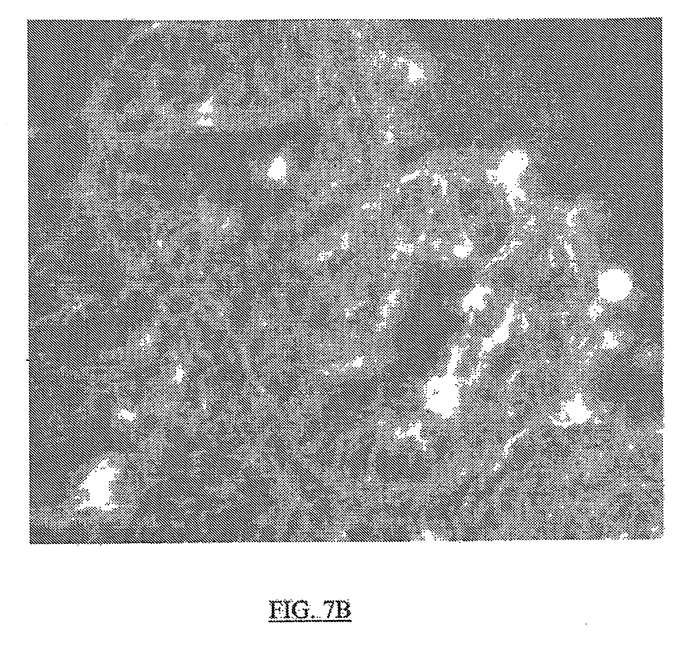

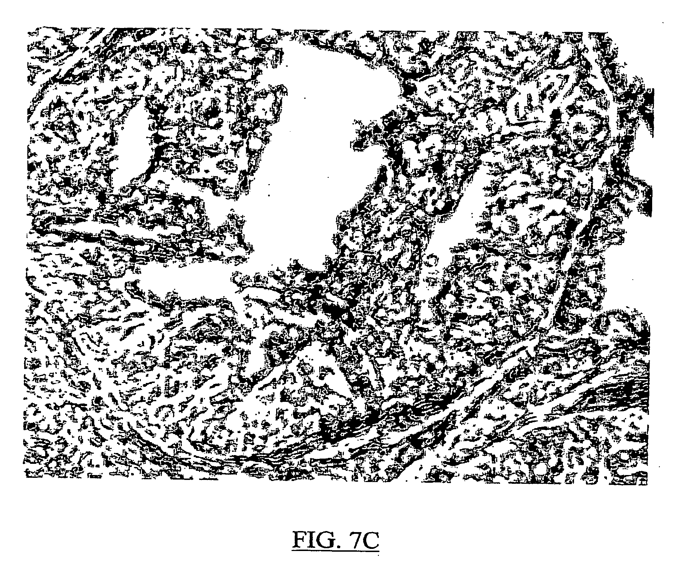

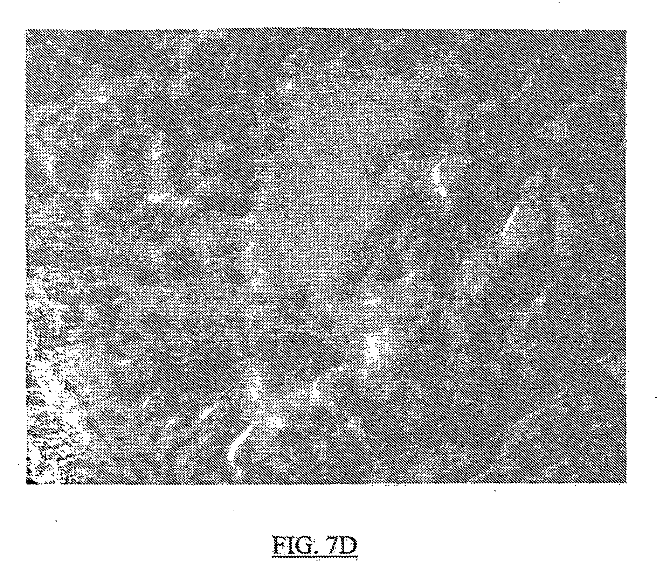

FIGS. 7A-7D include pictures of prostate sections of 20-week-old TRAMPxFVB F1 mice (Hybrid Mouse Prostatic Lumens) injected with 8 mg/kg_{BW} hydroethidine (HEt) i.v. one hour before sacrifice (wherein TRAMP is the TRansgenic Adenocarcinoma of the Mouse Prostate model), whereby FIG. 7A shows a representative phase contrast microscopic picture of prostate section from animals treated with vehicle only, whereby FIG. 7B shows a fluorescence microscopic picture of the prostate section shown in FIG. 7A, whereby FIG. 7C shows a representative phase contrast microscopic picture of the prostate section from animals sacrificed 2 weeks after treatment with 6 administrations of 25 mg/kg_{BW} of the N,N'-bis(2,3-butadienyl)-1,4-butanediamine compound, whereby FIG. 7D shows a HEt fluorescence microscopic picture of the prostate section shown in FIG. 7C, whereby all pictures were taken at 20x magnification using an Olympus™ BH-2 fluorescence microscope using 480 nm excitation/600 nm emission filters coupled with a Sony DSC-V3 digital camera set at F2.8 and 30 sec, whereby the pictures illustrate a reduction in oxidative stress due to administration of the drug, and whereby less HEt fluorescence indicates less oxidative stress in mice treated with MDL 72,527.

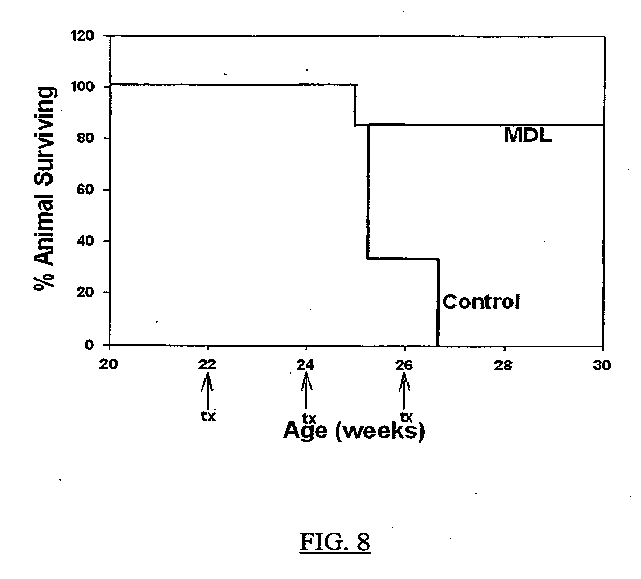

FIG. 8 is a graphical illustration of a plot of % animal surviving vs. age of TRAMP male mice, whereby the animals were treated with 25 mg/kg_{BW} of the N,N'-bis(2,3-butadienyl)-1,4-butanediamine compound or saline vehicle control i.p. on a bi-weekly regimen (at weeks marked "tx") and followed for survival, whereby survival was determined by the time to euthanization due to tumor burden, and whereby the data shows an improvement in overall survival by treating with MDL 72,527 in TRAMP mice.

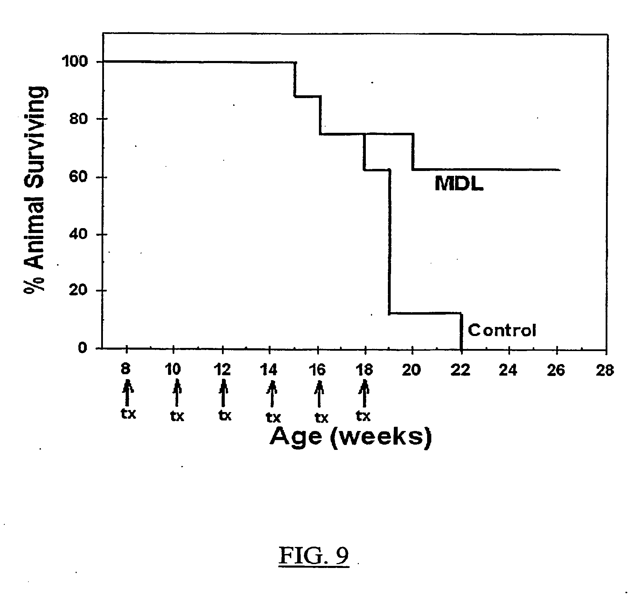

FIG. 9 is a graphical illustration of a plot of animal body weight (g) vs. animal age (weeks) for TRAMPxFVB mice treated with 25 mg/kg_{BW} of MDL 72,527 or saline vehicle and a control on a bi-weekly regimen (at weeks marked "tx"), whereby the data demonstrates that the MDL 72,527 has no material toxic effect as determined by changes in body weight of the TRAMPxFVB mice.

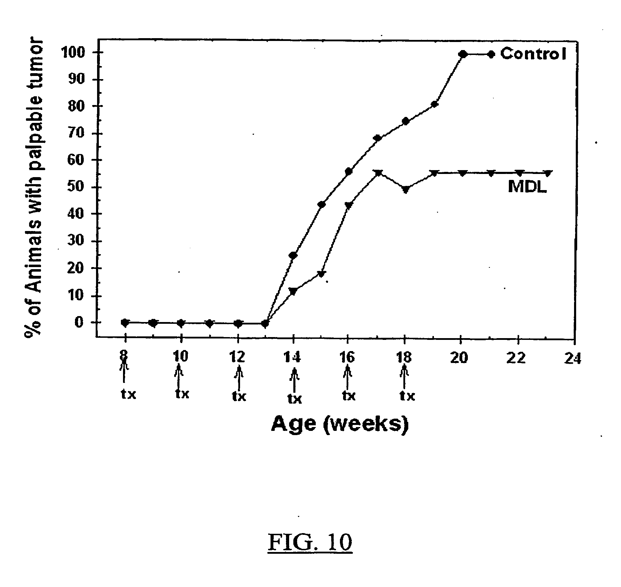

FIG. 10 is a graphical illustration of a plot of percent animals with distinct palpable tumor vs. animal age (weeks), whereby TRAMPxFVB mice were treated with 25 mg/kg_{BW} of the N,N-bis(2,3-butadienyl)-1,4-butanediamine compound every two weeks (at "tx"), and whereby the data demonstrates that the MDL 72,527 treatment delays time to tumor development in TRAMPxFVB mice.

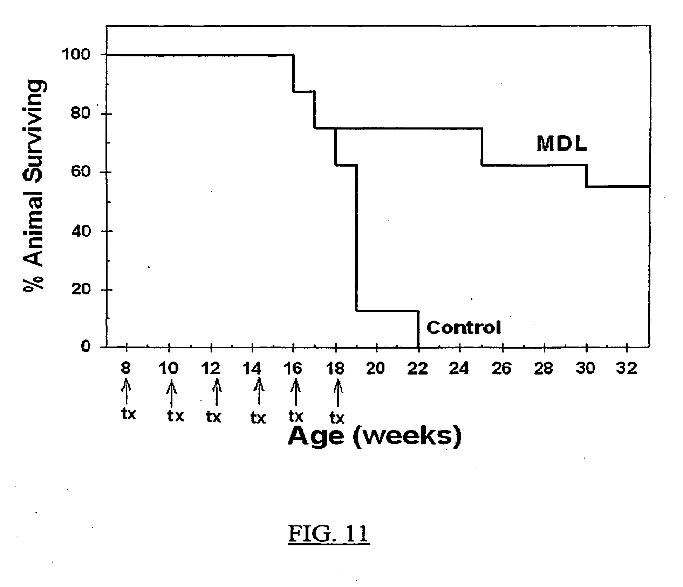

FIG. 11 is a graphical illustration of a plot of % animal surviving vs. age (weeks) of TRAMPxFVB male mice, whereby the animals were treated with 25 mg/kg_{BW} of the N,N-bis(2,3-butadienyl)-1,4-butanediamine compound or saline vehicle control i.p. on a bi-weekly regimen (at weeks marked "tx") and followed for survival, whereby survival was determined by the time to euthanization due to tumor burden, and whereby the data shows an improvement in overall survival by treating with MDL 72,527 in TRAMPxFVB mice.

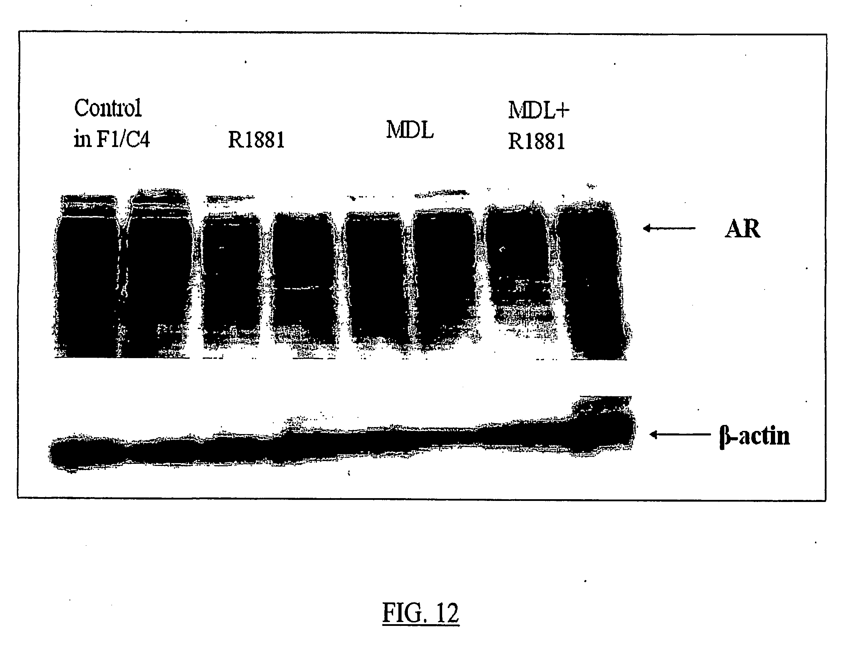

FIG. 12 is a Western blot analysis showing that administration of the N,N-bis(2,3-butadienyl)-1,4-butanediamine compound induced blockage of the androgen effect in TRAMPxFVB mice was not due to down-regulation of the Androgen Receptor ("AR").

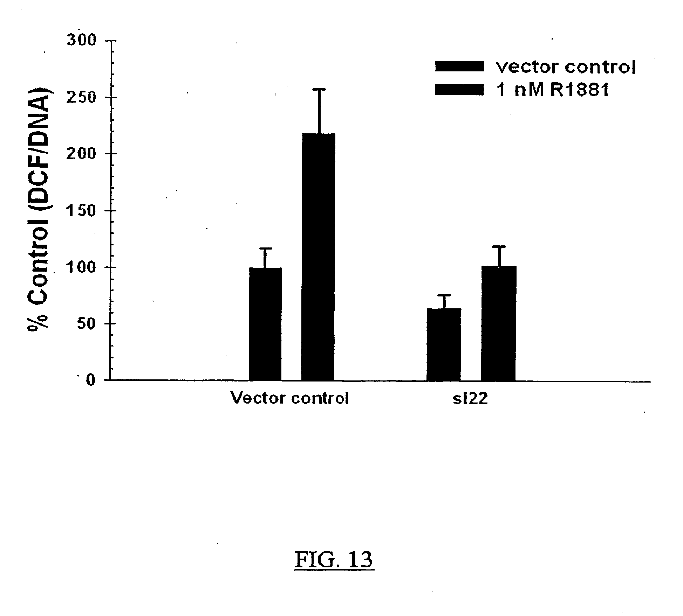

FIG. 13 shows a plot of DCF fluorescence/DNA fluorescence representing ROS levels in LNCaP cell clones transfected with vector alone or with vector expressing siRNA against SSAT (si22), whereby cells were untreated or treated with 1 nM R1881 for 96 h, whereby all data were normalized to that of untreated cells transfected with vector alone, whereby the 1 nM R1881 induced ROS production was markedly reduced in SSAT-silenced LNCaP clone cells (si22), and whereby the data show that polyamine oxidation is the source of ROS in PCa.

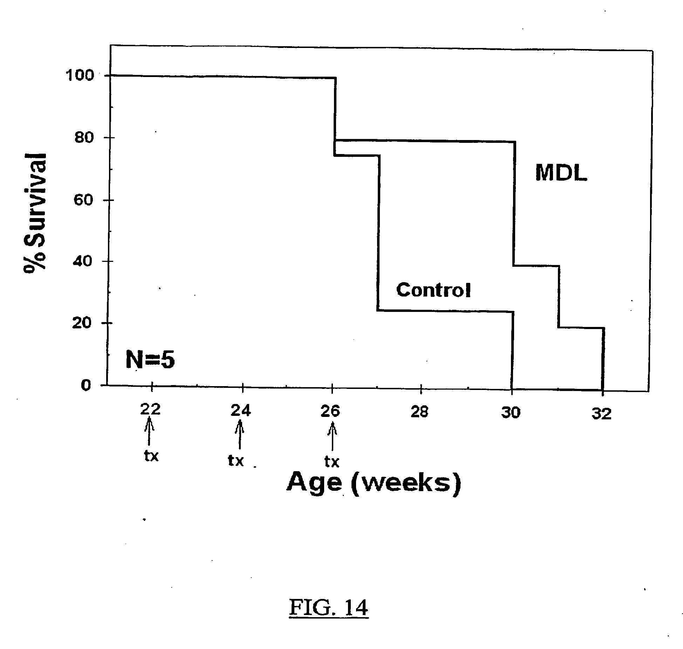

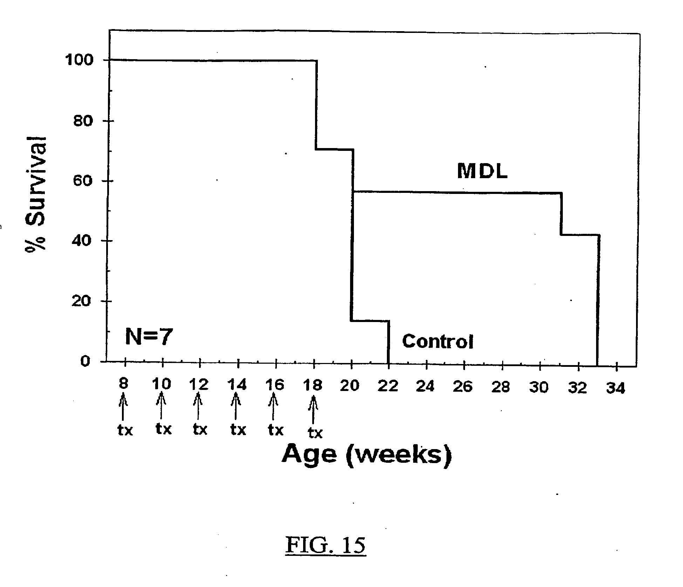

FIGS. 14 and 15 show Kaplan-Meier survival plots of percent survival vs. age of TRAMP in FIG. 14 and TRAMPxFVB male mice in FIG. 15, whereby the animals were treated with either 25 mg/kg_{BW} MDL 72,527 or saline vehicle control by intraperitoneal injection bi-weekly at weeks marked by arrows and followed for survival, whereby survival was determined by the time to euthanization due to tumor burden, whereby (as shown in FIG. 14) MDL 72,527 that was administered once in two weeks x3 treatment improved overall survival of TRAMP animals, and whereby (as shown in FIG. 15) MDL 72,527 that was administered one in 2 weeks x6 treatment increased overall survival of TRAMPxFVB mice.

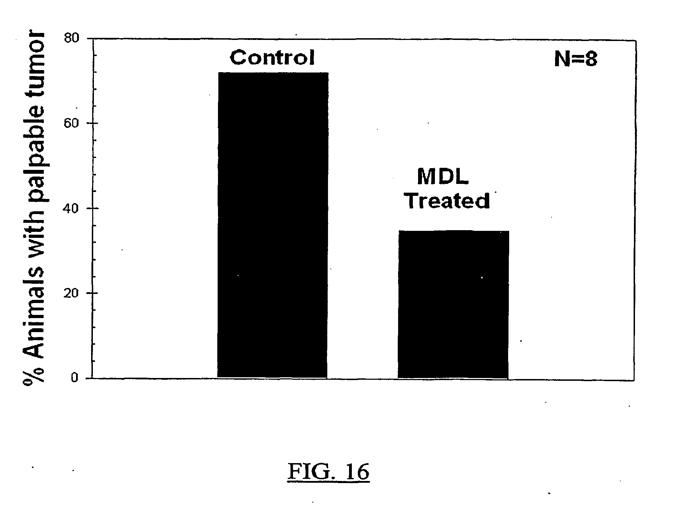

FIG. 16 shows a plot of twenty-week old animals with palpable tumor before 8 of the animals were sacrificed for microscopic determination of existence of Prostatic Intraepithelial Neoplasm (PIN) or carcinoma in prostate tissue by pathological examination, whereby the time to tumor development in TRAMPxFVB treated with MDL 72,527 at 25 mg/kg_{BW} once every two weeks is shown.

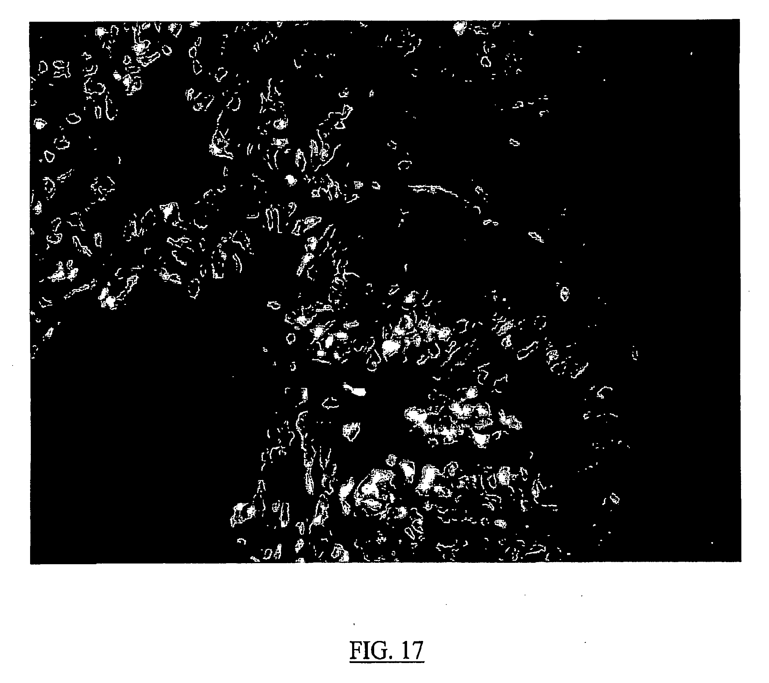

FIG. 17 shows a picture of surgically removed human prostatic lumen tissue from untreated men sliced in 4-5 mm thickness and incubated in HEt (8mg/ml) solution for 60 min. at 37°C before tissue processing (paraffin blocking and microtome sectioning for fluorescence micrography), whereby pictures were taken in 20x magnification at 480 nm excitation/595 nm emission wavelength, after treatment which included.

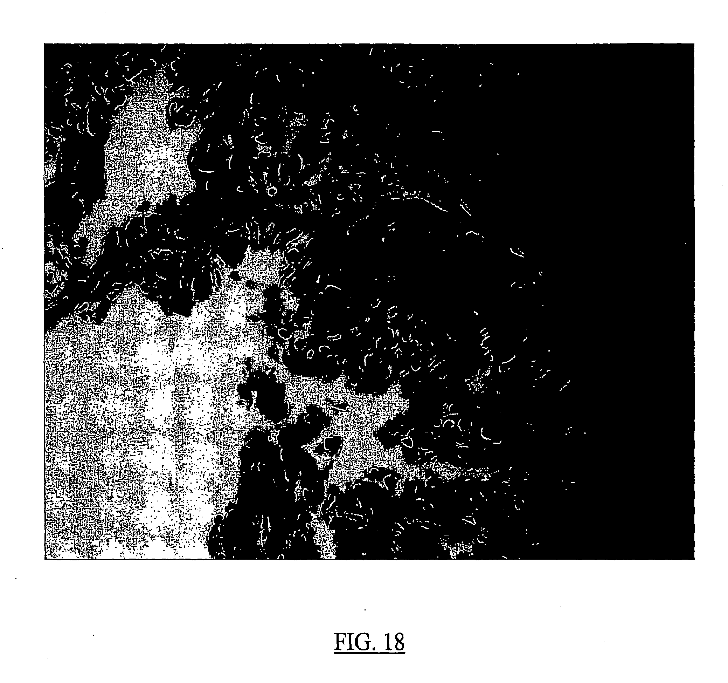

FIG. 18 shows a picture of the same section of human prostate lumen used in FIG. 17, whereby the HEt staining shows a difference between the epithelial cells and the stromal cells, and whereby the data confirm high oxidative stress only in the prostatic epithelial cells.

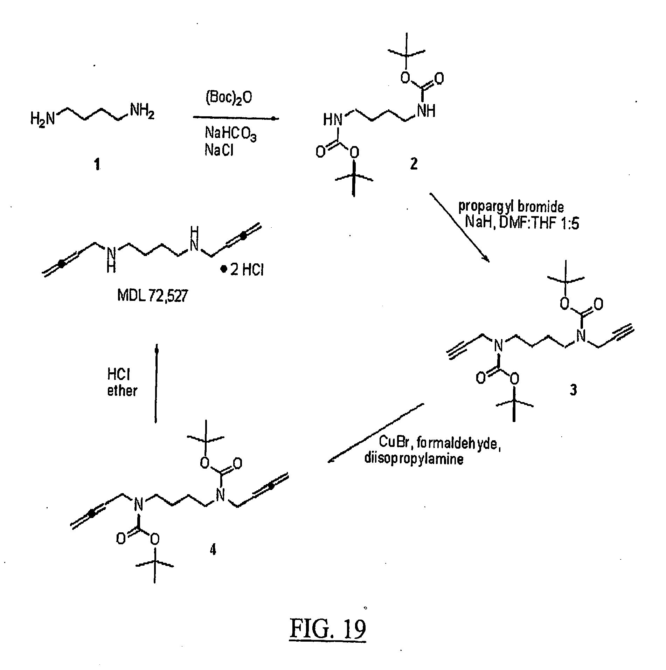

FIG. 19 shows the instant method of making N,N'-bis(2,3-butadienyl)-1,4-butanediamine.

### DETAILED DESCRIPTION OF THE PREFERRED EMBODIMENTS

The invention is directed to N1,N4-bis(buta-1,3-dienyl)butane-1,4-diamine dihydrochloride (also referred to as MDL 72,527 and N,N'-di-2,3-butadienyl-1,4-butanediamine dihydrochloride), or salts or solvates thereof, its use in preventing and/or treating prostate cancer in male humans, for example by reducing the concentration of reactive oxygen species in human prostate gland tissue or any other body tissue, and methods of making the compound thereof. Other uses include treating human males prostate cancer by inhibiting acetyl polyamine oxidase in human prostate tissue or other human body tissue. The invention may be used *ex vivo* or *in vivo* on any mammal such as a human or a dog.

The instant invention includes chemotherapeutic agents that specifically reduce oxidative stress in prostate tissue thereby preventing and/or treating prostate cancer progression, particularly in high-risk patients. It has been established that reactive oxygen species ("ROS") are produced in the prostate gland at relatively high levels as compared to other organs. ROS include, but are not limited to, hydrogen peroxide, superoxide, hydroxyl radical and nitric acid.

Without being limited to any theory, it is theorized that administration of the instant N,N'-bis(2,3-butadienyl)-1,4-butanediamine compound inhibits the acetyl polyamine oxidase ("APAO") and polyamine oxidase (PAO) processes by specifically blocking the biochemical pathway for androgen-induced oxidative stress (i.e., production of ROS) and thereby targeting antioxidant therapy toward prostate gland tissue. It is further theorized that administration of the instant N,N'-bis(2,3-butadienyl)-1,4-butanediamine compound has no adverse material effect on the androgen-signaling pathway. The structure of NN'-bis(2,3-butadienyl)-1,4-butanediamine is shown below. 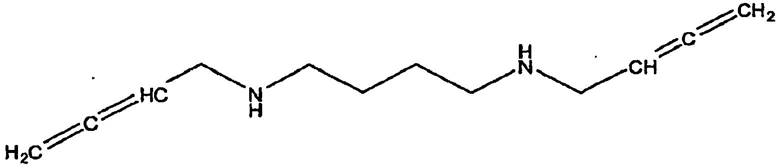 Preferably, a free-form, salt or solvate of N,N'-bis(2,3-butadienyl)-l,4-butanediamine is administered to a human or non-human mammal. The salt or solvate form may be any pharmaceutically suitable salt or solvate. Preferably, the pharmaceutically suitable salt form is N,N'-bis(2,3-butadienyl)-1,4-butanediamine •2HCl. Preferably, the pharmaceutically suitable solvate form is N,N'-bis(2,3-butadienyl)-1,4-butanediamine •2HCl dissolved in a pharmaceutically suitable solvent, such as a polar solvent, preferably water.

As such, administration of the instant N,N'-bis(2,3-butadienyl)-1,4-butanediamine compound successfully delayed prostate tumor development and increased the overall survival rate in the well-accepted, preclinical transgenic adenocarcinoma of mouse prostate ("TRAMP") model. (See, e.g., Garcia, GE et al, 2-Methoxyestradiol Inhibits Prostate Tumor Development in Transgenic Adenocarcinoma of Mouse Prostate: Role of Tumor Necrosis Factor-α-Simulated Gene 6, Clin Cancer Res 12(3) (1Feb2006). The TRAMP model spontaneously develops prostate cancer and dies from the disease. Administration of the instant N,N-bis(2,3-butadienyl)-1,4-butanediamine compound markedly reduced oxidative stress in a cultured, androgen dependent human tumor cell line and in preneoplastic lesions in the TRAMP animal *in vivo.* The instant N,N'-bis(2,3-butadienyl)-1,4-butanediamine compound may also be administered as an adjuvant therapy to prevent recurrences in patients previously treated for primary prostate tumor.

It has been reported that ROS are produced in the prostate gland at a higher levels than other organs. ROS alters growth or apoptosis-related genes either by direct mutagenic effects on the DNA or by alterations in gene expression. High ROS levels in prostate tissue may play a major role in both initiation and progression of prostate cancer. ROS may cause lipid peroxidation, alter the activity ofthiol-dependent enzymes, or damage DNA.

Low levels of ROS act as mitogens inducing tumor or redox alterations due to ROS production play a key role in specific signal transduction pathways. High fat diets increase lipid peroxidation (thereby producing ROS) which cause a relatively higher incidence of prostate cancer in industrialized nations as compared to developing countries. Published data supports a decrease in the incidence of prostate cancer where the diet includes dietary antioxidants such as β-carotene, β-lycopene, vitamin E and selenium, which reduces cellular ROS levels.

Recently, experimental and clinical evidence directly links increased oxidative stress with increased development of prostate tumors. Immunohistochemistry has been used to measure oxidative stress induced oxidative damage to DNA bases in archival paraffin blocks of surgically removed malignant and normal human prostate tissues. Malignant and metastatic human prostate tumor tissues have shown higher ROS induced protein and DNA base modifications than normal prostate tissue. Immunohistochemistry has also shown that oxidative damage to DNA and protein is significantly higher in the preneoplastic lesions in the TRAMP prostate as compared to adjacent normal prostate tissue.

Androgen has been identified as a natural agent that induces oxidative stress in prostate tissue. Hydroethidine dye fluoresces upon oxidation by ROS. The presence of high oxidative stress in LNCaP human tumor xenografts has been observed in male nude mice *in vivo.* That increased level of oxidative stress in tumor-bearing mice was reduced within 72 hours after surgical castration removing the naturally occurring source of androgen.

Exact molecular mechanisms concerning androgen-induced production of ROS in prostate tissue is unknown. Other pathways leading to increased ROS production in CaP cells have been reported such as: Expression of nuclear transcription factors (like hypoxia-induced transcription factor ("HIF-1α"), NF-κB, AP-1, etc.); and, suppression of glutathione S-transferees expression leading to reduced levels of total glutathione, a reducing agent. The suggested pathways may not be mutually exclusive.

Spermidine and spermine are polyamines whereby the precursor diamine putrescine is an organic cation present in all mammalian cells. Such polyamines are essential for cell growth and proliferation. The semen of healthy men contains large amounts of spermine (~ 3 mM) produced primarily during prostatic secretion.

As shown in Fig. 1, polyamine catabolism is driven by a spermidine/spermine N-acetyltransferase ("SSAT") process that produces N-acetyl polyamines. N-acetyl polyamines are oxidized by constitutive enzyme APAO. APAO uses FAD that is reduced to FADH₂ during oxidation of the acetyl polyamines. FADH₂ is recycled back to FAD regenerating the active APAO enzyme through production of H₂O₂ (i.e., a ROS). Enhanced expression of polyamine catabolic enzymes (through induction of specific transcription factors and lowering of total cellular glutathione) may enhance cellular oxidative stress due to polyamine catabolism.

As shown in Fig. 1, SSAT is the rate-limiting enzyme of the polyamine catabolic pathway. Acetyl polyamines produced by SSAT function as substrates for APAO oxidation and concomitant ROS production. Recent DNA microarray and qRT-PCR data suggest that androgen induces 30-50-fold over-expression of the SSAT gene in androgen-dependent LNCaP human prostate cancer cells.

It is well-accepted in the art that androgen, at physiological concentrations, induces ROS production in androgen dependent prostate cancer cells. A 2',7'-dichlorodihydrofluorescein diacetate (DCF) oxidation assay was used to measure the ratio of oxidized DCF:DNA fluorescence which is an accepted measure of ROS levels in prostate cell lines. The data and results are shown in Table 1.

**Table 1**

| **ROS in prostate cells by DCFH oxidation assay** | | | | |
|---|---|---|---|---|
| **Cell Line** | **Medium** | **Treatment** | **DCF/DNA Fluor units** | **s.d.** |
| Immortalized prostatic epithelial cells | Ham's F12+F(5) | None | 0.09 | ±0.02 |
| DU-145 | DMEM+F(5) | None | 0.33 | ±0.03 |
| DU-145 | DMEM+F(5) | 1 µM BE-3-3-3 | 0.67 | ±0.07 |
| DU-145 | DMEM+F(5) | 1 µM BE-3-3-3 + 25 µM MDL 72,527 | 0.26 | ±0.02 |
| LNCaP | DMEM+F(5) | None | 0.99 | ±0.03 |
| LNCaP | DMEM+(F1/C4) | None | 1.20 | ±0.02 |
| LNCaP | DMEM+(F1/C4) | 1 nM R1881 | 3.34 | ±0.05 |
| LNCaP | DMEM+(F1/C4) | 1 nM R1881 + 15 µM Vitamin E | 1.80 | ±0.04 |
| LNCaP | DMEM+(F1/C4) | 1 µM BE-3-3-3 | 3.19 | ±0.04 |
| LNCaP | DMEM+(F1/C4) | 1 µM BE-1 µM BE-3-3-3 + 25 µM MDL 72,527 | 1.87 | ±0.04 |

| | | | | |
|---|---|---|---|---|
| F(5) = 5% F etal bovine serum F1/C4 = 1% Fetal bovine serum+ 4 % Charcoal stripped serum (androgen depleted) | | | | |

The LNCaP human prostate cancer cells were grown in an androgen-depleted medium of 1% FBS and 4% charcoal stripped FBS, (F1/C4) and in the presence or absence of 1 nM synthetic androgen analog R1881 with or without 15 µM antioxidant α-tocopherol (vitamin E) pretreatment. The ROS levels of LNCaP and DU-145 human prostate cancer cells are shown as being treated with BE-3-3-3, which is a known SSAT inducing agent, with or without 25 µM treatment with MDL 72,527.

The data in Table 1 suggest that ROS levels in all prostate cancer cell lines are relatively higher than those levels observed in normal prostatic epithelial cells. The data in Table 1 also suggests that LNCaP cells have relatively more ROS than DU-145 cells; that androgen analog R1881 at 1 nM concentration, which is comparable to physiological levels of androgen, enhances the ROS level in LNCaP cells; that a sublethal dose of BE-3-3-3 (1 µM) enhances the ROS levels in both cell lines; and that the ROS level enhancement is reversed/reduced/prevented by pretreatment with vitamin E and/or MDL 72,527.

As shown schematically in Fig. 1, unusually high polyamine levels in prostate cells and high induction of SSAT may induce large increases in ROS levels. A DNA microarray analysis of gene expression in control untreated LNCaP cells and cells treated with 1nM androgen analog R1881 for 96 hours was performed twice using Affymetrix Genechip Arrays. In both experiments, SSAT was clearly identified as being highly over-expressed in R1881 treated with LNCaP cells as compared to untreated control cells. Among the list of genes that are over-expressed (more than 10-fold over the control), SSAT was the only enzyme connected to the ROS-generating pathway.

Data generated using the DNA microarray and qRT-PCR is shown in Fig. 3. Each data point is an average of readings from qty. six identically-treated wells repeated twice in triplicates. The qRT-PCR data show a 50-fold increase in the SSAT gene expression of R1881 treated with LNCaP cells as compared to the untreated control cells. Its further noted that SSAT was over-expressed only in cells treated with 1nM R1881 (which induces oxidative stress) but not in cells treated with 0.05 nM R1881 where no increase in oxidative stress was observed. It can be thus postulated that androgen-induced SSAT gene expression is a significant contributor to cellular ROS production in androgen-dependent prostate cancer cells.

Fig. 4 shows the role of SSAT in cellular ROS production using bisethylnorspermine (BE-3-3-3). BE-3-3-3 is known to induce SSAT enzyme activity in various cell lines including androgen-dependent LNCaP and androgen-independent DU-145 prostate cancel cells. DU-145 cells were treated with increasing concentrations of BE-3-3-3 for 72 hours. Oxidative stress was measured using 96-well plate based 2'7'-dichlorfluorescein diacetate dye ("DCF") oxidation assay. Each data point is an average of qty. 6 identically-treated wells repeated twice in triplicates. The data demonstrates that BE-3-3-3 (at a non-cytotoxic dose, <1µM) increases polyamine oxidation and increases cellular ROS levels, and that polyamine oxidation is a significant factor for producing oxidative stress in prostatic cells.

Figs. 5A-5E show the effect of pre-treating LNCaP cells (with and without being treated with R1881) with the instant N,N'-bis(2,3-butadienyl)-1,4-butanediamine compound on polyamine and acetyl polyamine levels. The HPLC method of quantitation was used to quantify polyamine and acetyl polyamine levels in LNCaP cells treated with 1 nM R1881 ± 25 µM MDL 72,527. Each data point is an average of 2 determinations of cell pellets collected from qty. 3 independent experiments. Figs. 5A-5E show that treatment with R1881 (at a final concentration of 1 nM for 96 hours) significantly increases putrescine and spermidine levels, decreases the spermine level, and, increases the N-acetyl spermidine and N-acetyl spermine levels.

It can be stated that Figs. 5A-5E support the conclusion that R1881 treatment increases SSAT mRNA levels while enhancing SSAT enzyme activity. In R1881 cells, pretreatment with the instant N,N'-bis(2,3-butadienyl)-1,4-butanediamine compound almost completely blocked the induced increase in putrescine and spermidine levels (attributable to the 1 nM R1881), and it caused significant increase in N-acetyl-spermidine and N-acetylspermine levels without appreciably changing the spermine level. An insignificantly small increase in N-Ac-spermine level in cells treated with the instant N,N'-bis(2,3-butadienyl)-1,4-butanediamine compound was observed. It was also observed that cells treated with MDL 72,527 at 25 µM efficiently and effectively blocked APAO activity. The observed large increase in acetyl polyamine levels also suggests that administration of MDL 72,527 has no material effect on SSAT gene expression and/or enzymatic activity in androgen-treated cells.

Shown in Fig. 6 is data demonstrating that pretreatment administration of 25 µM MDL 72,527 effectively blocks androgen induced production of ROS in LNCaP cells. ROS levels in LNCaP cells were induced by increasing concentration of R1881 for 96 hours with and without the MDL 72,527 pretreatment. Results were determined using DCF oxidation assay methods. Each data point is an average of readings from qty. 6 wells repeated twice in triplicates. Data concerning the ROS levels of cells pre-treated with the N,N'-bis(2,3-butadienyl)-1,4-butanediamine compound were normalized respecting the effects of administering MDL 72,527 alone. The data in Fig. 6 suggest that pretreatment using MDL 72,527 (≥1 nM) effectively blocks R1881-induced ROS production. The data also suggest that inhibition of polyamine oxidase using MDL 72,527 significantly reduces cellular ROS levels in androgen-dependent prostate cancer cells, and that MDL 72,527 is an effective antioxidant that specifically blocks androgen-induced ROS production in androgen-dependent human prostate cancer cells.

Figs. 7A-7D show that administration of the N,N'-bis(2,3-butadienyl)-1,4-butanediamine compound reduces oxidative stress in prostate tissue in TRAMPxFVB animals *in vivo.* A method using hydroethidine (HEt) dye oxidation was standardized to observe oxidative stress in prostate tumors *in vivo.* HEt oxidizes to 2-hydroxy ethidium specifically by a ROS(41). The 2-hydroxy ethidium fluoresces at 488 nm excitation and 595 nm emission frequencies. The HEt dye may be safely injected into animals at least 1 hour before sacrifice. Twenty-week old TRAMPxFVB animals were injected with HEt (8 mg/kg_{BW}) 1 hour before sacrifice. After sacrifice, prostate glands were collected, formalin-fixed and paraffin-embedded. Paraffin blocks were microtome-sectioned, and the tissues were deparaffinized, observed using fluorescence microscopy, and analyzed using digital image analysis. The images are shown in Figs. 7A-7D).

Fig. 7A shows a phase contrast microscopic picture of a prostate section from animals treated with vehicle only. Fig. 7B shows fluorescence microscopic picture of the prostate section shown in Fig. 7A. Fig. 7C shows the phase contrast microscopic picture of the prostate section from animals sacrificed 2 weeks after treatment with 6 administrations of 25 mg/kg_{BW} MDL 72,527. Fig. 7D shows a HEt fluorescence microscopic picture of the prostate section shown in Fig. 7C. The picture shown in Figs. 7A-7D were taken at 20x magnification using an Olympus BH-2 fluorescence microscope using 480 nm excitation/600 nm emission filters coupled with a Sony DSC-V3 digital camera set at F2.8 and 30 sec. exposure.

High fluorescence (due to HEt oxidation) was generally observed in the prostatic lumen, particularly at the edges of the invading cells, which started forming prostatic intraepithelial neoplasia (PIN). In contrast, no dye oxidation was detected in the prostatic TRAMP mice tissues treated with MDL 72,527 (see Fig. 7D) as confirmed by the subsequent H&E staining. The data shown in Figs. 7A-7D suggest that the N,N-bis(2,3-butadienyl)-1,4-butanediamine compound significantly inhibits oxidative stress *in vivo* within the prostatic lumen of TRAMP mice that started forming PIN, which further suggests that polyamine oxidation is a significant contributor to oxidative stress in cultured human prostate cells and the prostatic lumen of TRAMP animals *in vivo.*

DCFH oxidation assay. The 96-well cultures or freshly re-sected animal or human tissues were assayed for estimation of ROS levels in intact cells using the dye 2',7' -dichlorofluorescein diacetate (DCF) (Molecular Probes, Inc., Eugene, OR). Re-sected tissues or cell cultures were washed with 200 µL Kreb's Ringer buffer (116 mM NaCl, 4.2 mM KCl, 2.5 mM CaCl₂, 1.6 mM NaH₂PO₄, 1.2 mM MgSO₄, 22 mM NaHCO₃, and 11 mM D-glucose), pre-warmed to 37°C, incubated at 37°C in 100 µL Kreb's Ringer buffer containing 10 µg/mL (final concentration) DCF dye for 45 minutes. Each 96-well culture plate was scanned on a CytoFluor 2350™ plate scanner (Applied Biosystems, Foster City, CA) using the 485 excitation/530 emission frequencies. Each tissue sample was scanned.

Hydroethidine Assay. Hydroethidine dye was dissolved in DMSO (100 mg/ml) and diluted in isotonic saline to 1 mg/ml before injection. The dye was either injected i.v. into mice through tail vein 1 hour before sacrifice (*in vivo*) or freshly re-sected human and animal tissues were washed in isotonic PBS and soaked in 8 mg/ml dye solution in Kreb's Ringer buffer pre-warmed at 37°C for 60 minutes at 37°C (*ex vivo*) before processing. For *in vivo* assays, animals were euthanized and bled before collecting tumor and other tissues. Tissues after treatment were embedded in paraffin blocks. Microtome sections of the blocks were mounted on slides and were quantitated for fluorescence at 488 nm excitation/ 595 nm emission frequencies.

DNA Assay. Fifty uL of Kreb's Ringer Buffer (40ug/mL) containing Hoechst 33342 dye, which contains 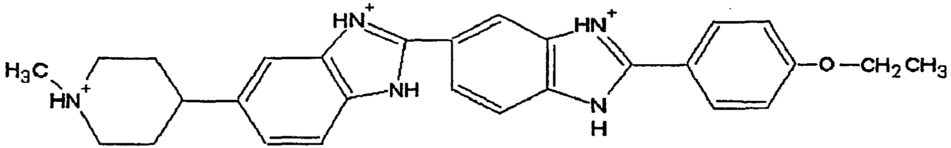 , was added to each well 45 minutes and to all tissues 60 minutes before fluorescence measurements. The DNA fluorescence was determined 360 nm excitation/460 nm emission. All DCF or Hydroethidine fluorescence were normalized to DNA fluorescence for proper quantitation of oxidative stress.

BD Bioimager. All fluorescence readings were quantified using a BD Pathway Bioimager automated, confocal, real-time, single cell kinetic and endpoint imaging system (BD Biosciences (Laguna Hills, CA).

The data shown in Fig. 8 suggest that administration of the N,N'-bis(2,3-butadienyl)-1,4-butanediamine compound increases the overall survival ("OS") rate of TRAMP mice. TRAMP mice that spontaneously form prostate tumors at 20-22 weeks of age and mostly die of the tumor burden at 30-32 weeks in age were tested. TRAMPxFVB mice that start forming prostate tumors at 12-14 weeks in age and mostly die of the tumor burden at 20-22 weeks in age were also tested (data shown in Figs. 9-11). As shown in Fig. 9, a dose of 25 mg/kg_{BW} of the MDL 72,527 was used, which was well-tolerated (i.e., no overt signs of toxicity, abnormal behavior or body weight loss was observed). A dose above 20 mg/kg_{BW} was required to completely inhibit mouse acetyl polyamine oxidase.

In the TRAMP animal study, the N,N'-bis(2,3-butadienyl)-1,4-butanediamine compound was administered at 22 weeks, whereby a few of the animals had started showing palpable tumors. The animals (qty. 5 in each group) were injected 3 times with 25 mg/kg_{BW} of MDL 72,527 i.p. on a bi-weekly regimen. OS is shown in Fig. 8, which suggests significant improvement compared to animals treated with the vehicle.

In the study using the TRAMPxFVB mice, qty. 8 animal were in the MDL 72,527-treated group, and qty. 8 mice were in the vehicle-treated group. Since these animals start prostate tumor development at an earlier age than do the TRAMP animals, treatment began at 8 weeks of age. Treatment included qty. 6 injections of MDL 72,527 at 25 mg/kg_{BW} i.p. on a bi-weekly administration regimen schedule. The results are shown in Fig. 11 which suggests a significant increase in OS for the animals treated with MDL 72,527 as compared to the vehicle control. Over 60% of the MDL 72,527-treated TRAMPxFVB mice survived at least 8 weeks after the end of the therapy, and at least 6 weeks after the death of the entire group of control vehicle-treated animals. The data in Figs. 8 and 9 show that different strains of TRAMP animals reproducibly show over 60% survival benefit for MDL 72,527-treated animals as compared to the vehicle-treated mice.

The data shown in Fig. 10 suggest that administration of the N,N'-bis(2,3-butadienyl)-1,4-butanediamine compound delays the time to tumor development in TRAMPxFVB mice. Signs of palpable prostate tumor development were monitored twice every week. At 11 weeks (29 week old animals) after MDL 72,527-treatment (at 25 mg/kg_{BW}, administered once bi-weekly) was discontinued, over 40% of the treated mice exhibited no signs of palpable tumor in the prostate or in any other part of the body.

The data shown in Figs. 1-18 demonstrate that the N,N'-bis(2,3-butadienyl)-1,4-butanediamine compound effectively blocks androgen induced oxidative stress in human prostate cancer cells and in the prostate gland of TRAMP animals. The data also show that MDL 72,527 delays time to tumor development in TRAMP animals resulting in a statistically significant increase in OS.

Another aspect of the invention is an improved synthesis of making N,N'-bis(2,3-butadienyl)-1,4-butanediamine. MDL 72,527 is a potent, irreversible inhibitor of mammalian polyamine oxidase and it exhibits a Kᵢ of 0.9 mM against pig liver polyamine oxidase. A known synthesis of MDL involves converting N-Boc-protected propargylamine to the corresponding (bis)allene, followed by coupling of 2 equivalents of the protected allene to 1,4-diiodobutane. Deprotection of the resulting N-Boc-protected (bis)allene yielded MDL 72,527. The known synthesis is problematic in that the electrophilic nature of the allene moiety yielded a number of side products during alkylation of the diiodide. The overall yield is disadvantageously low.

The instant method of making N,N'-bis(2,3-butadienyl)-1,4-butanediamine has an advantageously high yield. The instant method also avoids making undesirable side products. The instant method is set forth in Fig. 19.

Commercially available putrescine 1 is (bis)-N-Boc protected to produce compound 2 (85.2% yield). Compound 2 is used to alkylate qty. 2 equivalents of propargyl bromide in the presence of sodium hydride producing compound 3 (59.5% yield). Yields in that transformation are further enhanced by using a mixture of dimethylformamide ("DMF") and tetrahydrofuran ("THF") in a ratio of 1:5. The propargyl groups in compound 3 are converted to the corresponding allenes in the presence of CuBr, formaldehyde, and diisopropylamine to yield intermediate compound 4 (38.7% yield). Compound 4 is de-protected in the presence of HCl to yield the desired target molecule MDL 72,527 (white solid, 65.8% yield).

HPLC method of quantitation of MDL, natural polyamines and their acetyl derivatives in cell extracts as well as in human and animal serum is standardized. Therefore, the pharmacokinetics and pharmocodynamics of MDL 72,527 may be determined.

Salts of the instant N,N-bis(2,3-butadienyl)-1,4-butanediamine compound may be a pharmaceutically suitable (i.e., pharmaceutically acceptable) salt including, but not limited to, acid addition salts formed by mixing a solution of the MDL compound with a solution of a pharmaceutically acceptable acid. The pharmaceutically acceptable acid may be hydrochloric acid, methanesulphonic acid, fumaric acid, maleic acid, succinic acid, acetic acid, benzoic acid, oxalic acid, citric acid, tartaric acid, carbonic acid or phosphoric acid. Various pharmaceutically acceptable salts are well known in the art and may be used with N,N'-bis(2,3-butadienyl)-1,4-butanediamine such as those disclosed in (Berge SM et al., "Pharmaceutical Salts." J. Pharm. Sci. 66:1-19 (1977) and Haynes DA et al., "Occurrence of pharmaceutically acceptable anions and cations in the Cambridge Structural Database," J. Pharm. Sci. 94:2111-2120 (2005), which are hereby incorporated herein by reference. For example, the list of FDA-approved commercially marketed salts includes acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methylsulfate, mucate, napsylate, mitrate, pamoate, pantothenate, phosphate, diphosphate, polygalacturonate, salicylate; stearate, subacetate, succinate, sulfate, tannate, tartrate, teoclate, and triethiodide.

The N,N'-bis(2,3 butadienyl)-1,4-butanediamine compound (or salt or solvate thereof) may be administered as an oral dosage form such as uncoated tablets, coated tablets, hard or soft gelatin capsules, powders, capsules, pellets, solutions, suspensions, elixirs or emulsions. The oral dosage form may be administered in accordance with a dosing regimen to achieve a suitable therapeutic effect. The oral dosage form is also defined by a pharmaceutical composition comprising the pharmaceutical active ingredient (API) and various pharmaceutically acceptable/suitable carriers (aqueous, non-aqueous, solutions, suspensions, or emulsions), excipients, solvents, additives, vehicles, stabilizers, inert diluents, binders (e.g., acacia, cornstarch, gelatin), disintegrating agents (e.g., cornstarch, potato starch, alginic acid), lubricants (e.g., stearic acid, magnesium stearate) and the like.

For example, pharmaceutically acceptable aqueous carriers include, but are not limited to, gums, starches, sugars, lactose, sucrose, cellulosic materials, water, alcohol/water mixtures, phosphate buffer (0.01-0.1M, or more preferably 0.05M) and 0.9% saline. Non-aqueous solvents include, but are not limited to, propylene glycol, polyethylene glycol, vegetable oil (e.g., olive oil, ethyl oleate, and the like). Various pharmaceutically acceptable USP approved excipients may also be used, including but not limited to, albumin, gelatin, detergents (e.g., Tween 20, Tween 80, Pluronic F68, bile acid salts and the like), solubilizing agents (e.g., glycerol, polyethylene glycerol, and the like), antioxidants (e.g., ascorbic acid, sodium metabisulfite, and the like), preservatives (e.g., thimerosal, benzyl alcohol, paraben, and the like), fatty acids, waxes, poloxamers, poloxamines, bulking substances or tonicity modifiers (e.g., lactose, mannitol, and the like), polymers for covalent attachment or complexation with metal ions, polylactic acid, polyglycolic acid, hydrogel agents, liposomes, microemulsion agents, micelle agents, milamellar agents, multilamellar vesicles, erythrocyte ghost agents or spheroplast agents.

## Claims

1. A compound which is N,N'-bis(2,3-butadienyl)-1,4-butanediamine or a pharmaceutically suitable salt or solvate thereof for use as a monotherapy in the treatment of cancer in the prostate of a human male.

2. A compound which is N,N'-bis(2,3-butadienyl)-1,4-butanediamine or a pharmaceutically suitable salt or solvate thereof for use as a monotherapy in the prophylactic treatment of cancer in the prostate of a human male.

3. A compound which is N,N'-bis(2,3-butadienyl)-1,4-butanediamine or a pharmaceutically suitable salt or solvate thereof for use as a monotherapy in the treatment of cancer in the prostate of a male non-human mammal.

4. A compound which is N,N'-bis(2,3-butadienyl)-1,4-butanediamine or a pharmaceutically suitable salt or solvate thereof for use as a monotherapy in the treatment of cancer in the prostate of a human male by inhibition of acetyl polyamine oxidase.

5. A compound as defined in any preceding claims, wherein the salt is a dihydrochloride salt.

6. A compound as defined in any preceding claims, which is used in a therapeutic amount sufficient to stop or reduce the progression, morbidity and/or mortality due to prostate cancer.

7. A compound as defined in claim 6, wherein the therapeutic amount is in the range of about 1-100 mg/kg_{BW}, and wherein the therapeutic amount is dosed in the range of bi-weekly to daily.

8. A compound as defined in claim 7, wherein the therapeutic amount is in the range of about 10-40 mg/kg_{BW}, and wherein the therapeutic amount is dosed weekly.

9. A compound as defined in claim 7, wherein the therapeutic amount is around 25 mg/kg_{BW}, and wherein the therapeutic amount is dosed bi-weekly.

10. The compound of claim 4, wherein the acetyl polyamine oxidase is inhibited by at least 50% as compared to an untreated human male.

## Patentansprüche

1. Eine Zusammensetzung zur Verwendung als Monotherapie bei der Behandlung von Prostatakrebs bei einem männlichen Menschen, die N,N'-bis(2,3-Butadienyl)-1,4,Butandiamin oder ein pharmazeutisch verträgliches Salz oder Solvat dieses ist.

2. Eine Zusammensetzung zur Verwendung als Monotherapie bei der prophylaktischen Behandlung von Prostatakrebs bei einem männlichen Menschen, die N,N'-bis(2,3-Butadienyl)-1,4,Butandiamin oder ein pharmazeutisch verträgliches Salz oder Solvat dieses ist.

3. Eine Zusammensetzung zur Verwendung als Monotherapie bei der Behandlung von Prostatakrebs bei einem männlichen, nicht-menschlichen Säugetier, die N,N'-bis(2,3-Butadienyl)-1,4,Butandiamin oder ein pharmazeutisch verträgliches Salz oder Solvat dieses ist.

4. Eine Zusammensetzung zur Verwendung als Monotherapie bei der Behandlung von Prostatakrebs bei einem männlichen Menschen durch Hemmung von Acetylpolyaminoxidase, die N,N'-bis(2,3-Butadienyl)-1,4,Butandiamin oder ein pharmazeutisch verträgliches Salz oder Solvat dieses ist.

5. Eine Zusammensetzung entsprechend einem der vorangehenden Ansprüche, wobei das Salz ein Dihydrochlorsalz ist.

6. Eine Zusammensetzung entsprechend einem der vorangehenden Ansprüche, die in einer ausreichenden therapeutischen Menge verwendet wird, um die Progression, Morbidität und/oder Mortalität infolge von Prostatakrebs zu stoppen oder zu reduzieren.

7. Eine Zusammensetzung entsprechend Anspruch 6, wobei die therapeutische Menge im Bereich von ca. 1-100 mg/kg_{KG} liegt und wobei die therapeutische Menge im Bereich von alle zwei Wochen bis täglich dosiert wird.

8. Eine Zusammensetzung entsprechend Anspruch 7, wobei die therapeutische Menge im Bereich von ca. 10-40 mg/kg_{KG} liegt und wobei die therapeutische Menge wöchentlich dosiert wird.

9. Eine Zusammensetzung entsprechend Anspruch 7, wobei die therapeutische Menge ca. 25 mg/kg_{KG} beträgt und wobei die therapeutische Menge alle zwei Wochen dosiert wird.

10. Die Zusammensetzung entsprechend Anspruch 4, wobei die Acetylpolyaminoxidase im Vergleich zu einem nicht behandelten männlichen Menschen um mindestens 50% gehemmt wird.

## Revendications

1. Un composé qui est N,N'-bis(2,3-butadiényle)-1,4-butanediamine ou un sel pharmaceutiquement acceptable ou un solvant de celui-ci pour une utilisation en tant que monothérapie dans le traitement du cancer de la prostate chez l'homme de sexe masculin.

2. Un composé qui est N,N'-bis(2,3-butadiényle)-1,4-butanediamine ou un sel pharmaceutiquement acceptable ou un solvant de celui-ci pour une utilisation en tant que monothérapie dans le traitement prophylactique du cancer de la prostate chez l'homme de sexe masculin.

3. Un composé qui est N,N'-bis(2,3-butadiényle)-1,4-butanediamine ou un sel pharmaceutiquement acceptable ou un solvant de celui-ci pour une utilisation en tant que monothérapie dans le traitement du cancer de la prostate chez un mammifère mâle non humain.

4. Un composé qui est N,N'-bis(2,3-butadiényle)-1,4-butanediamine ou un sel pharmaceutiquement acceptable ou un solvant de celui-ci pour une utilisation en tant que monothérapie dans le traitement du cancer de la prostate chez l'homme de sexe masculin par une inhibition de l'oxydase de polyamine acétyle.

5. Un composé selon l'une quelconque des Revendications précédentes, où le sel est un sel de dihydrochlorure.

6. Un composé selon l'une quelconque des Revendications précédentes, qui est utilisé dans une quantité thérapeutique suffisante pour arrêter ou réduire la progression, la morbidité et/ou la mortalité due au cancer de la prostate.

7. Un composé selon la Revendication 6, où la quantité thérapeutique est dans la plage d'environ 1-100 mg/kg_{BW}, et où la quantité thérapeutique est un dosage dans la plage de bihebdomadaire à quotidien.

8. Un composé selon la Revendication 7, où la quantité thérapeutique est dans la plage d'environ 10-40 mg/kg_{BW}, et où la quantité thérapeutique est un dosage hebdomadaire.

9. Un composé selon la Revendication 7, où la quantité thérapeutique est d'environ 25 mg/kg_{BW}, et où la quantité thérapeutique est un dosage bihebdomadaire.

10. Le composé selon la Revendication 4, où l'oxydase de polyamine acétyle est inhibée d'au moins 50% en comparaison d'un homme de sexe masculin non traité.
